# FASCICULE DE BREVET EUROPEEN

(11) **EP 1 354 593 B1**
(45) Date de publication et mention de la délivrance du brevet: **02.03.2011**
(21) Numéro de dépôt: 03007876.0
(22) Date de dépôt: 07.04.2003
(51) Int. Cl.: A61K 35/74

(54) **Utilisation d'un extrait de bacterie filamenteuse non photosynthetique non fructifiante en tant qu'agent augmentant la synthese endogene de superoxyde dismutase**
Verwendung von einem Extrakt von einem nicht-photosynthetischen faserigen Bakterium zur Beförderung der Herstellung von Superoxid-dismutase
use of an extract of a non-photosynthetic filamentous bacterium as an agent for enhancing the endogenous synthesis of superoxide dismutase

(30) Priorité: 08.04.2002 FR 0204339
(43) Date de publication de la demande: 22.10.2003
(73) Titulaire: L'Oréal, 75008 Paris (FR)
(72) Inventeur: Mahe, Yann, 91390 Morsang Sur Orge (FR); Meybeck, Alain, 92400 Courbevoie (FR)
(74) Mandataire: Allab, Myriam

(56) Documents cités:
- EP-A- 0 761 204
- EP-A- 1 022 018
- WO-A1-02/056858
- FR-A- 2 693 654
- FR-A- 2 719 768
- FR-A- 2 819 407
- US-A- 5 618 521

## Description

La présente invention se rapporte à l'utilisation cosmétique d'un extrait de bactérie filamenteuse non photosynthétique non fructifiante dans une composition renfermant un milieu cosmétiquement acceptable, en tant qu'agent augmentant la synthèse endogène de superoxyde dismutase (SOD), pour prévenir et/ou limiter la formation de radicaux libres et/ou éliminer les radicaux libres présents dans les cellules.

Au cours du temps, il apparaît différents signes sur la peau, très caractéristiques du vieillissement, se traduisant notamment par une modification de la structure et des fonctions cutanées.

Ce vieillissement de nature physiologique résulte de l'action de deux grandes classes de composantes, une composante endogène résultant notamment de la production naturelle d'ions superoxydes, en particulier produits lors de la respiration cellulaire.
L'autre composante est exogène. En effet, le vieillissement peut être accéléré par des facteurs environnementaux tels qu'une exposition répétée de la peau à la lumière solaire, et notamment aux rayonnements ultraviolets A, à la pollution, notamment aux particules de diesel et à la fumée de cigarette.
On sait que la toxicité des polluants atmosphériques, notamment des polluants gazeux tels que le dioxyde de soufre, l'ozone et les oxydes d'azote sur les constituants de la peau (fibres, cellules, enzymes) et sur le sébum sécrété par la peau est liée notamment à leur activité d'initiateurs de radicaux libres, source de phénomènes d'oxydation qui provoquent chez les êtres vivants des dommages cellulaires.
Les cellules vivantes, qui sont en contact direct et permanent avec le milieu extérieur (notamment la peau, le cuir chevelu et certaines muqueuses) sont particulièrement sensibles à ces effets des polluants gazeux, qui se traduisent notamment par un vieillissement accéléré de la peau, avec un teint manquant d'éclat et une formation précoce de rides ou ridules, et aussi par une diminution de la vigueur et un aspect terne des cheveux.
On sait également que les phénomènes d'irritation provoqués par l'exposition aux rayons ultra-violet conduisent aussi au phénomène de vieillissement cellulaire accéléré.

Qu'ils aient une origine endogène ou exogène, les radicaux libres provoquent des dégâts oxydatifs importants, notamment dans les membranes cellulaires (peroxydation de lipides provoquant une dégradation de la perméabilité des membranes), les noyaux des cellules (destruction de l'ADN), et les tissus, en particulier le tissu conjonctif (dégradation des fibres d'élastine et de collagène, dépolymérisation des fibres polyuroniques). Ces dégâts conduisent notamment à un dessèchement et à une perte de fermeté et d'élasticité de la peau (Grinwald et al. 1980, Agren et al. 1997).

Les spécialistes considèrent actuellement qu'une des causes du vieillissement cellulaire est l'amoindrissement des capacités de défense contre les radicaux libres et contre les phénomènes d'oxydation (notamment la formation d'ions superoxyde) qu'ils initient.

L'ion superoxyde O°-2 (oxygène actif) est un ion-radical dont l'instabilité et la réactivité en font un composé toxique, car il engendre, notamment en présence d'ions métalliques, des radicaux libres hydroxyle (OH°) hautement nocifs. Les superoxyde dismutases (SOD) sont des enzymes qui exercent un effet protecteur notamment en piégeant les ions superoxydes et constituent ainsi un système biologique de défense contre les effets nocifs des radicaux libres.

Les superoxyde dismutases sont capables d'induire la dismutation des ions superoxydes, suivant la réaction : 2O^{°-}₂ + 2H⁺ → H₂O₂ + O₂

On connaît de nombreuses superoxyde dismutases. Par exemple, on a déjà décrit des superoxyde dismutases extraites d'érythrocytes de boeuf (Markovitz, J. Biol. Chem. 234, p. 40, 1959) et des superoxyde dismutases extraites d'*Escherichia coli* (Keele et Fridovich, J. Biol. Chem., 245, p. 6176, 1970). Dans le document FR-A-2.225.443, sont décrites des superoxyde dismutases extraites de souches bactériennes marines, ainsi que leur procédé de préparation.

Les superoxyde dismutases permettent en particulier de protéger la peau et les cheveux, notamment en maintenant l'intégrité de la structure kératinique naturelle, comme le décrit par exemple le document FR-A-2.287.899. En outre, les superoxyde dismutases améliorent la respiration cellulaire cutanée et maintiennent ou améliorent les qualités de la peau telles que la douceur au toucher, la souplesse et l'élasticité.

Les superoxyde dismutases protègent aussi la peau contre les phénomènes d'inflammation causés par les rayonnements ultra-violets ainsi que contre le vieillissement accéléré de la peau notamment sous l'influence de tels rayonnements.

En raison de ces propriétés intéressantes, il est connu d'ajouter des superoxyde dismutases dans des compositions cosmétiques, en particulier des compositions destinées à une application topique (voir, par exemple, les demandes de brevet EP 0 673 643 et EP 0 636 360).

La Demanderesse a découvert un nouveau moyen de lutter contre les effets néfastes provoqués par les radicaux libres en induisant la synthèse endogène de superoxyde dismutase. En effet, elle a découvert de façon tout à fait surprenante qu'un extrait de bactérie filamenteuse non photosynthétique et non fructifiante provoquait l'augmentation de la synthèse endogène de superoxyde dismutase.

L'utilisation topique de substances à activité superoxyde dismutase, si elle peut être avantageuse dans certaines conditions d'utilisation, présente néanmoins l'inconvénient de nécessiter une stabilité et une biodisponibilité cutanée particulières de ces formes SOD. Par ailleurs, on sait qu'il existe une distribution particulière des SOD dans les cellules, notamment elles sont physiologiquement localisées à l'intérieur des cellules et plus particulièrement à proximité directe des entités cellulaires fortement génératrices d'ions superoxydes que sont les mitochondries. Ainsi, lorsqu'on utilise des moyens de neutraliser les ions superoxydes produits à l'extérieur de la cellule, c'est que les dégâts cellulaires oxydatifs sont déjà très importants et que l'anion superoxyde est sorti du contrôle de son environnement intracellulaire. L'utilisation de SOD exogène a donc pour mission d'empêcher la propagation des dégâts oxydatifs à d'autres entités cellulaires pouvant remplir une fonction à la fois curative et réparatrice.

La Demanderesse présente ici un moyen d'induire les cellules à produire leur propre activité antioxydante et à renforcer ainsi leurs défenses naturelles. L'avantage de l'utilisation selon la présente invention par rapport à l'utilisation d'une substance exogène présentant une activité SOD est qu'elle permet, après contact des cellules avec l'extrait membranaire de bactérie filamenteuse non photosynthétique non fructifiante, d'induire la production d'enzyme SOD selon une localisation (notamment mitochondriale) qui respecte les conditions physiologiques et surtout induit la cellule à se protéger de l'intérieur grâce à son propre métabolisme oxydatif. De plus, la SOD, lorsqu'elle capture des radicaux libres, subit elle même une oxydation et perd progressivement sa résistance aux dommages oxydatifs, ceci est encore plus manifeste lorsque l'enzyme est sortie de son contexte intracellulaire. L'induction de SOD en réponse à l'application d'un extrait de bactérie filamenteuse non photosynthétique non fructifiante permet ainsi à la cellule d'auto-reconstituer et d'accélérer le renouvellement physiologique de son stock intracellulaire de SOD.

Augmenter la synthèse endogène de superoxyde dismutase présente de nombreux avantages par rapport à l'apport exogène de l'enzyme. En effet, lors de l'application topique de compositions contenant des SOD, une proportion importante de SOD sont dénaturées notamment par des protéases présentes à la surface de la peau et/ou ne franchissent pas les membranes cellulaires en raison de leur poids moléculaire important (17kD pour la Cu/ZnSOD et 23 kD pour la MnSOD). Aussi, pour obtenir l'effet désiré, il est généralement nécessaire d'augmenter la quantité de SOD dans la composition.
Un autre inconvénient de l'application topique de protéine exogène, telle que l'enzyme SOD, est le risque d'allergie qu'elle représente lorsque la concentration en protéine est trop élevée.

La présente invention a donc pour objet l'utilisation cosmétique d'un extrait de bactérie filamenteuse non photosynthétique non fructifiante dans une composition renfermant un milieu cosmétiquement acceptable, en tant qu'agent augmentant la synthèse endogène de superoxyde dismutase.

Les extraits de bactéries selon l'invention sont préparés à partir de bactéries filamenteuses non photosynthétiques et non fructifiantes telles que définies selon la classification du Bergey's Manual of Systematic Bacteriology (vol. 3, section 23, 9°édition, 1989), parmi lesquelles on peut citer les bactéries appartenant à l'ordre des Beggiatoales, et plus particulièrement les bactéries appartenant aux genres Beggiatoa, Vitreoscilla, Flexithrix ou Leucothrix.

Différents extraits pourront être utilisés, en particulier, on préférera utiliser comme extrait de bactérie filamenteuse non photosynthétique non fructifiante un isolat de lipopolysaccharides qui pourra, par exemple, être obtenu selon l'une des méthodes décrites dans l'exemple 1.

La bactérie filamenteuse non photosynthétique non fructifiante préférée selon la présente invention est *Vitreoscilla filiformis*, en particulier, la souche ATCC 15551.

Les cellules humaines synthétisent deux types de superoxyde dismutase, la superoxyde dismutase de type 1, aussi appelée Cu/ZnSOD ou SOD du cytosol, est une enzyme qui se trouve dans le cytosol des cellules. La superoxyde dismutase de type 2, MnSOD, se retrouve dans les mitochondries des cellules. La Demanderesse a constaté de façon tout à fait surprenante que l'extrait de bactérie filamenteuse non photosynthétique non fructifiante était particulièrement efficace pour augmenter la synthèse de MnSOD par une voie transcriptionnelle impliquant l'augmentation de production de l'ARN messager, précurseur de sa synthèse protéique.

Aussi une variante de l'invention se rapporte à l'utilisation cosmétique d'un extrait de bactérie filamenteuse non photosynthétique non fructifiante dans une composition renfermant un milieu cosmétiquement acceptable, en tant qu'agent augmentant la synthèse endogène de superoxyde dismutase de type 2 (MnSOD).

Dans les compositions selon l'invention, on peut utiliser 0,001 à 10%, et en particulier de 0,01 à 1%, en poids d'extrait sec de bactérie filamenteuse non photosynthétique non fructifiante par rapport au poids de la composition.
Dans des formes d'applications particulières de type balneothérapie, on peut également envisager des applications de lysats bactériens natifs ou reconstitués en des proportions supérieures pouvant atteindre 100%.

Ces compositions peuvent contenir l'extrait de *Vitreoscilla filiformis* sous forme de dispersion dans un véhicule approprié tel que, par exemple, l'eau, les solvants organiques, les corps gras y compris les huiles, et leurs mélanges, notamment des émulsions.

Selon un autre objet de la présente invention et pour toutes les applications décrites ci-après, lors de l'utilisation cosmétique de l'extrait de bactérie filamenteuse non photosynthétique non fructifiante dans une composition renfermant un milieu cosmétiquement acceptable, l'extrait de bactérie filamenteuse non photosynthétique non fructifiante peut être associé à un agent antioxydant.

Ainsi, on peut par exemple utiliser un agent antioxydant choisi parmi :

La bactérie filamenteuse non photosynthétique non fructifiante préférée selon la présente invention est *Vitreoscilla filiformis,* en particulier, la souche ATCC 15551.

US 5.618.521 décrit une composition antisolaire comprenant un extrait de bactéries filamenteuses non Photosynthétiques et non fructifiantes associé à un agent antioxydant.

FR 2 693 654 et EP 1 022 018 décrivent l'utilisation de fractions lipopolysaccharidiques de bactéries filamenteuses non photosynthètiques et non fructifiantes respectivement comme immunostimulant pour diverses applications thérapeutiques parmi lesquelles certaines sont cutanées telles que brulures et plaies et, comme agent amincissant (effet lipolytique), dans des compositions cosmétiques topiques. II n'est ni évoqué, ni suggéré dans ces documents, la capacité de la fraction lipopolysaccharidique de ces bactéries à lutter contre des phénomènes oxydatifs indésirables au sein de la peau et du cuir chevelu.

Les cellules humaines synthétisent deux types de superoxyde dismutase, la superoxyde dismutase de type 1, aussi appelée Cu/ZnSOD ou SOD du cytosol, est une enzyme qui se trouve dans le cytosol des cellules. La superoxyde dismutase de type 2, MnSOD, se retrouve dans les mitochondries des cellules. La Demanderesse a constaté de façon tout à fait surprenante que l'extrait de bactérie filamenteuse non photosynthétique non fructifiante était particulièrement efficace pour augmenter la synthèse de MnSOD par une voie transcriptionnelle impliquant l'augmentation de production de l'ARN messager, précurseur de sa synthèse protéique.

Aussi une variante de l'invention se rapporte à l'utilisation cosmétique d'un extrait de bactérie filamenteuse non photosynthétique non fructifiante dans une composition renfermant un milieu cosmétiquement acceptable, en tant qu'agent augmentant la synthèse endogène de superoxyde dismutase de type 2 (MnSOD).

Dans les compositions selon l'invention, on peut utiliser 0,001 à 10%, et en particulier de 0,01 à 1%, en poids d'extrait sec de bactérie filamenteuse non photosynthétique non fructifiante par rapport au poids de la composition.
Dans des formes d'applications particulières de type balneothérapie, on peut également envisager des applications de lysats bactériens natifs ou reconstitués en des proportions supérieures pouvant atteindre 100%.
. la vitamine E (tocophérol) et ses dérivés, entre autre acétate, linoléate ou nicotinate, de préférence à des concentrations de l'ordre de 0,1 à 5%,
. le γ-orizanol (0,1 à 5%),
. les pidolates de lysine ou d'arginine (0,5 à 10%),
. les extraits végétaux tels que l'extrait de mélisse (0,01 à 2%), l'extrait de silimarine (0,01 à 2%), l'extrait de gingko biloba (0,05 à 2%), l'extrait de sauge (0,05 à 2%), l'extrait de noix de cola (0,05 à 2%), l'extrait de rutine (0,1 à 2%) ou l'extrait de thym (0,1 à 2%), les % étant donnés en matière sèche,
. le lycopène sous une forme purifiée ou bien dans un extrait (par exemple pâte de tomate titrés en lycopene aboutissant à une concentration finale de lycopène comprise entre 10⁻⁻⁷% et 10% et plus préférentiellement de 10⁻⁷% à 0,1%),
. les oligomères proanthocyanolidiques de pin, d'aubépine ou de raisin (0,1 à 2%),
. le di-tertiobutyl-hydroxybenzylidène camphre (0,1 à 2%),
. le thé vert (0,1 à 2%),
. la caféine (0,1 à 5%),
. le glycérol (2 à 30%),
. le mannitol (2 à 30%),
. la carnosine (0,1 à 2%),
. la superoxyde dismutase (100 à 10 000 UI/100 g),
. la guanosine (0,01 à 1%),
. les microalgues contenant de l'ethoxyquine telles que l'Hématococcus (0,005 à 1%),
. le pentasodium aminotriméthylène phosphonate (0,001 à 0,5%),
. la lactoperoxydase (0,01 à 0,1%) et
. la lactoferrine (0,01 à 0,1%).
On peut également utiliser un mélange de plusieurs agents antioxydants.
On peut également citer des agents anti-radicalaires, en particulier, les bioflavonoïdes ; le co-enzyme Q10 ou ubiquinone ; certaines enzymes comme la catalase, le glutathion peroxydase et les quinones réductases ; le glutathion ; le benzylidène camphre ; les benzylcyclanones ; les naphtalénones substituées ; les pidolates ; le phytantriol ; les lignanes ; et la mélatonine.
De préférence, l'agent antioxydant est le lycopène.

Dans un autre mode de réalisation selon l'invention, l'agent antioxydant est une superoxyde dismutase. On pourra par exemple utiliser l'enzyme SOD extraite d'érythrocytes de boeuf (Markovitz, J. Biol. Chem. 234, p. 40, 1959), d'*Escherichia coli* (Keele et Fridovich, J. Biol. Chem., 245, p. 6176, 1970) ou encore, de souches bactériennes marines (FR-A-2.225.443).

La présente invention se rapporte également à l'utilisation cosmétique d'un extrait de bactérie filamenteuse non photosynthétique non fructifiante dans une composition renfermant un milieu cosmétiquement acceptable, pour prévenir et/ou limiter la formation de radicaux libres et/ou éliminer les radicaux libres présents dans les cellules.

Un autre objet de l'invention se rapporte à l'utilisation cosmétique d'un extrait de bactérie filamenteuse non photosynthétique non fructifiante dans une composition renfermant un milieu cosmétiquement acceptable, pour prévenir et/ou lutter contre les effets néfastes des UV et/ou de la pollution sur la peau.

Cliniquement, les effets néfastes des UV et/ou de la pollution sur la peau se traduisent généralement par un vieillissement accéléré, c'est-à-dire par l'apparition de rides et ridules, par un relâchement des tissus cutanés et sous-cutanés, par une perte de l'élasticité cutanée et par une atonie de la texture de la peau. La perte de fermeté et de tonicité de la peau, comme les rides et les ridules, s'explique au moins en partie par une atrophie dermique et ainsi qu'un aplanissement de la jonction dermo-épidermique ; la peau est moins ferme et plus flasque, et l'épaisseur de l'épiderme diminue.
En outre, le teint de la peau est généralement modifié, il apparaît plus pâle et plus jaune. Ce phénomène semble dû essentiellement une désorganisation de la microcirculation (moins d'hémoglobine au niveau du derme papillaire). Un autre signe clinique du vieillissement est l'aspect sec et rêche de la peau qui est dû essentiellement à une desquamation plus importante ; ces squames, en diffractant les rayons lumineux, participent aussi l'aspect un peu gris du teint. De plus, de nombreuses taches colorées et/ou plus foncées apparaissent la surface de la peau, et plus spécialement sur les mains, conférant la peau une hétérogénéité. En général, ces taches sont dues à une production importante de mélanine dans l'épiderme et/ou le derme de la peau. Par ailleurs, il peut exister sur certaines zones de la peau des irritations diffuses et parfois des télangiectasies. Certains de ces signes sont plus particulièrement liés au vieillissement intrinsèque ou physiologique, c'est-à-dire au vieillissement lié à l'âge, alors que d'autres sont plus spécifiques du vieillissement extrinsèque, c'est-à-dire du vieillissement provoqué d'une manière générale par l'environnement ; il s'agit plus particulièrement du photo-vieillissement dû l'exposition au soleil, la lumière ou tout autre rayonnement.

Ainsi l'objet de l'invention est particulièrement adapté à l'utilisation cosmétique d'un extrait de bactérie filamenteuse non photosynthétique non fructifiante dans une composition renfermant un milieu cosmétiquement acceptable, pour prévenir et/ou traiter la perte de fermeté et/ou d'élasticité de la peau. Une telle utilisation permet notamment à la peau de retrouver un aspect uniformément lisse.

Un autre objet de l'invention est l'utilisation cosmétique d'un extrait de bactérie filamenteuse non photosynthétique non fructifiante dans une composition renfermant un milieu cosmétiquement acceptable, pour prévenir et/ou traiter le teint terne.

L'invention est également adaptée à l'utilisation cosmétique d'un extrait de bactérie filamenteuse non photosynthétique non fructifiante dans une composition renfermant un milieu cosmétiquement acceptable, pour prévenir et/ou traiter la déshydratation cutanée.

De façon plus générale, l'objet de l'invention est également adapté à l'utilisation cosmétique d'un extrait de bactérie filamenteuse non photosynthétique non fructifiante dans une composition renfermant un milieu cosmétiquement acceptable, pour prévenir et/ou traiter les signes du vieillissement cutané.

Par signes du vieillissement cutané, on entend plus particulièrement les taches pigmentaires et/ou les taches d'hyperkératose et/ou l'atrophie épidermique et/ou la rugosité cutanée et/ou la sécheresse cutanée.

Comme expliqué précédemment, un effet indésirable de la présence de radicaux libres dans la peau est qu'ils provoquent un phénomène de péroxydation des lipides. Avec l'âge (plus particulièrement à partir de quarante ans), l'accumulation de ces lipides péroxydés est responsable de mauvaises odeurs corporelles telles qu'une odeur rance (Haze S. et al. J. Invest. Dermatol. 2001, 116(4) : 520-4).

L'objet de l'invention est adapté à l'utilisation cosmétique d'un extrait de bactérie filamenteuse non photosynthétique non fructifiante dans une'composition renfermant un milieu cosmétiquement acceptable, pour prévenir et/ou limiter et/ou éliminer la péroxydation des lipides cutanés.
Ainsi l'objet de l'invention est également utile pour prévenir et/ou limiter et/ou éliminer les mauvaises odeurs corporelles.

Dans le cas particulier de l'exposition de la peau au soleil, il apparaît qu'une exposition modérée aux UVA et UVB induit dans un premier temps une diminution des SOD cutanées (Leccia et al., Exp. Dermatol. 2001, 10(4) : 272-9). Cinq jours après cette exposition, un effet rebond est constaté avec une élévation de l'activité des SOD. Ainsi, une période transitoire est nécessaire à la mise en place d'un système protecteur anti-oxydant cutané.
De par sa capacité à augmenter la synthèse endogène de SOD, l'extrait de bactérie filamenteuse non photosynthétique non fructifiante permet d'accélérer la mise en place du système protecteur anti-oxydant cutané et prépare la peau à une exposition solaire.

Aussi, l'utilisation cosmétique selon l'invention d'un extrait de bactérie filamenteuse non photosynthétique non fructifiante dans une composition renfermant un milieu cosmétiquement acceptable est particulièrement bien adaptée pour préparer la peau à une exposition solaire.

En particulier, la préparation de la peau à une exposition solaire pourra être faite par l'application quotidienne sur la peau de ladite composition cosmétique pendant une semaine avant l'exposition solaire et, de préférence, pendant deux semaines, jusqu'à au moins une nuit (entre 6 et 18 heures) avant l'exposition solaire.

L'utilisation selon la présente invention est également utile pendant et après l'exposition solaire pour maintenir un niveau élevé de synthèse de SOD et pour atténuer et/ou réparer les dommages liés à l'exposition solaire tels que les rougeurs, les irritations cutanées et les sensations d'échauffement de la peau.

L'objet de l'invention pourra également consister en l'utilisation cosmétique d'un extrait de bactérie filamenteuse non photosynthétique non fructifiante dans une composition renfermant un milieu cosmétiquement acceptable pour atténuer et/ou réparer les rougeurs et/ou les irritations cutanées et/ou les sensations d'échauffement de la peau provoquées par une exposition solaire.

De façon préférentielle, la composition cosmétique utilisée selon la présente invention est adaptée à une application topique.

L'utilisation d'un extrait de bactérie filamenteuse non photosynthétique non fructifiante dans une composition cosmétique selon l'invention pourra également se présenter sous la forme d'un tonique capillaire pour revitaliser le cuir chevelu et améliorer l'aspect de la chevelure.

Dans une autre variante de l'invention, l'utilisation d'un extrait de bactérie filamenteuse non photosynthétique non fructifiante dans une composition cosmétique est appliquée sur les ongles pour revitaliser les ongles. Dans une telle application, la composition cosmétique pourra par exemple se présenter sous la forme d'un vernis ou d'un gel ou d'une crème de massage pour les ongles.

Un autre objet de la présente invention se rapporte à l'utilisation d'au moins un extrait de bactérie filamenteuse non photosynthétique non fructifiante pour la préparation d'une composition dermatologique destinée à augmenter la synthèse endogène de superoxyde dismutase.

Ainsi l'utilisation d'au moins un extrait de bactérie filamenteuse non photosynthétique non fructifiante peut être également utile pour la préparation d'une composition dermatologique destinée à prévenir et/ou limiter la formation de radicaux libres et/ou éliminer les radicaux libres présents dans les cellules.

En particulier, la présente invention se rapporte à l'utilisation d'au moins un extrait de bactérie filamenteuse non photosynthétique non fructifiante pour la préparation d'une. composition dermatologique destinée à réparer les dégâts provoqués par une exposition solaire, en particulier, lorsque les dégâts provoqués par une exposition solaire sont un érythème cutané ou un oedème cutané.

La présente invention se rapporte également à l'utilisation d'au moins un extrait de bactérie filamenteuse non photosynthétique non fructifiante pour la préparation d'une composition dermatologique destinée à prévenir et/ou limiter et/ou éliminer les phénomènes d'oxydation provoqués par la colonisation de la peau par des microorganismes. Préférentiellement, l'invention se rapporte à l'utilisation d'au moins un extrait de bactérie filamenteuse non photosynthétique non fructifiante pour la préparation d'une composition dermatologique destinée à traiter l'acné.

Il a été montré que les tissus d'une peau psoriatique exprimaient de façon plus importante les ARNm codant pour la MnSOD et que ce phénomène représentait une réponse visant à protéger les cellules (Lontz et al., Free Radic. Biol. Med. 1995 ; 18(2) :349-55). Par l'augmentation de la synthèse endogène de MnSOD, l'utilisation selon l'invention contribue au traitement des peaux psoriatiques. Aussi l'objet de l'invention est adapté à l'utilisation d'au moins un extrait de bactérie filamenteuse non photosynthétique non fructifiante pour la préparation d'une composition dermatologique destinée à traiter le psoriasis.

Par ailleurs, une étude sur un modèle de carcinogenèse de la peau comparant l'évolution de tumeurs sur de souris transgéniques exprimant la MnSOD dans la peau et sur des souris sauvages a montré que l'expression de MnSOD avait pour conséquence l'inhibition du développement des tumeurs (Zhao et al., Cancer Res. 2001, 61(16) :6082-8). Aussi l'objet de l'invention est adapté à l'utilisation d'au moins un extrait de bactérie filamenteuse non photosynthétique non fructifiante pour la préparation d'une composition dermatologique destinée à inhiber le développement des tumeurs cancéreuses cutanées.

La présente invention se rapporte également à l'utilisation d'au moins un extrait de bactérie filamenteuse non photosynthétique non fructifiante pour la préparation d'une composition dermatologique destinée à prévenir et/ou soigner les signes du vieillissement épidermique. Ces signes du vieillissement cutané sont notamment les taches pigmentaires et/ou les taches d'hyperkératose et/ou l'atrophie épidermique et/ou la rugosité cutanée et/ou la sécheresse cutanée.

Dans une autre variante, la présente invention se rapporte à une composition renfermant, dans un milieu physiologiquement acceptable au moins un extrait de bactérie filamenteuse non photosynthétique non fructifiante, de préférence un extrait de *Vitreoscilla filiformis*, et du lycopène.

Le lycopène est un pigment naturel que l'on trouve dans les fruits mûrs, particulièrement dans la tomate. Il appartient à la famille des caroténoïdes et sa structure est proche de celle du β-carotène.
Le rôle du lycopène dans la maturation des fruits est connu dans l'art antérieur.
Le lycopène est utilisé dans des compositions à activité bronzante pour son rôle sur la synthèse de mélanine (WO 97/47278), dans des compositions destinées au traitement de la chevelure et/ou de l'acné pour son activité sur les 5α-réductases (JP-2940964) ou encore comme agent anti-radicalaire (JP-A-8-283136).

Le lycopène est également décrit comme inhibiteur de l'expression des protéases de la matrice extracellulaire, particulièrement des métalloprotéinases, comme par exemple les collagénases (EP-A-1090628).

Le lycopène peut être sous forme chimique cis ou trans.

Pour donner un ordre de grandeur, on peut utiliser le lycopène pur en une quantité représentant de 10⁻⁻⁷% à 0,1% du poids total de la composition et préférentiellement en une quantité représentant de 10⁻⁷% à 10⁻³% du poids total de la composition.
Pour donner un autre ordre de grandeur, certaines pâtes de tomates vendue dans le commerce telle que Lyc-O-Derm® (Lycored) sont titrées à 10% de lycopéne pur, on peut donc utiliser une quantité d'extrait pâteux de tomate dans des proportions allant de 10⁻⁶% à 10⁻²% d'extrait de tomate.
Dans les applications particulière de balneothérapie, on peut envisager un contact avec la peau dans des proportions où l'extrait de tomate peut-être appliqué pur et atteindre des titres de lycopène appliqués sur la peau ou les muqueuses pouvant aller de 1% à 15%.

encapsulation (voir par exemple les demandes PCT WO 01/46431 et WO 87/07838 et EP-A-0 397 227).
On peut utiliser notamment les lactoperoxydases, les microperoxydases de champignons, la myéloperoxydase, etc... On sait que la lactoperoxydase (en abrégé : LPO) est une enzyme que l'on trouve notamment dans de nombreux tissus et sécrétions de mammifères, qui utilise un des nombreux donneurs d'électrons cellulaires pour réduire les peroxydes organiques du type ROOH (R étant un groupement organique). La lactoperoxydase est un produit commercial, vendu notamment par les Sociétés Sigma et Sederma.
On peut également utiliser des peroxydases recombinantes, par exemple la LPO recombinante (demande de brevet WO 91/06639).

Enfin, la composition selon l'invention peut renfermer dans un milieu physiologiquement acceptable au moins un extrait de *Vitreoscilla filiformis,* un composé ayant une activité catalase et un composé ayant une activité peroxydase.

Les compositions utilisées selon l'invention peuvent se présenter sous toutes les formes envisageables dans le domaine cosmétique et, en particulier, dermatologique.

La composition selon l'invention est de préférence adaptée à une application topique sur la peau. Elle peut se présenter sous toutes les formes galéniques normalement utilisées pour ce type d'application, notamment sous forme d'une solution aqueuse ou huileuse, d'une émulsion huile-dans-eau ou eau-dans-huile ou multiple, d'une émulsion siliconée, d'une microémulsion ou nanoémulsion, d'un gel aqueux ou huileux ou d'un produit anhydre liquide, pâteux ou solide.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau.

Il peut même s'agir, dans les applications assimilées à la balnéothérapie, d'un extrait brut.

Pour renforcer les effets anti-âge de la composition selon l'invention, celle-ci peut renfermer, outre l'extrait de bactérie filamenteuse non photosynthétique non fructifiante décrit précédemment, au moins un composé choisi parmi : les agents desquamants et/ou hydratants; les agents dépigmentants ou propigmentants; les agents anti-glycation ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents myorelaxants ; les agents anti-pollution et/ou anti-radicalaire ; les agents amincissants ; les agents agissant sur la microcirculation ; les agents agissant sur le métabolisme énergétique des cellules ; les agents tenseurs ; et leurs mélanges.

Ainsi, la composition selon l'invention pourra notamment contenir au moins un actif choisi parmi : les α-hydroxyacides ; l'acide salicylique et ses dérivés tels que l'acide n-octanoyl-5-salicylique ; l'HEPES ; la procystéine ; l'O-octanoyl-6-D-maltose; le sel disodique d'acide méthyl glycine diacétique ; les céramides ; les stéroïdes tels que la diosgénine et les dérivés de la DHEA ; l'acide kojique ; le N-éthyloxycarbonyl-4-para-aminophénol ; l'acide ascorbique et ses dérivés ; les extraits de myrtille ; les rétinoïdes et en particulier le rétinol et ses esters ; les polypeptides et leurs dérivés acylés ; les phytohormones ; les extraits de levure *Saccharomyces cerevisiae* ; les extraits d'algues ; les extraits de soja, de lupin, de maïs et/ou de pois ; l'alvérine et ses sels, en particulier le citrate d'alvérine ; le resvératrol ; les caroténoïdes et en particulier le lycopène ; le tocophérol et ses esters ; le co-enzyme Q10 ou ubiquinone ; les xanthines et en particulier la caféine et les extraits naturels en contenant ; les extraits de petit houx et de marron d'Inde ; et leurs mélanges, sans que cette liste soit limitative.

La composition selon l'invention peut en outre contenir au moins un filtre UVA et/ou UVB. Les filtres solaires peuvent être choisis parmi les filtres organiques, les filtres inorganiques et leurs mélanges.

Comme exemples de filtres organiques actifs dans l'UV-A et/ou l'UV-B, on peut citer notamment, désignés ci-dessous par leur nom CTFA :
- les dérivés de l'acide para-aminobenzoïque : PABA, Ethyl PABA, Ethyl Dihydroxypropyl PABA, Ethylhexyl Diméthyl PABA vendu notamment sous le nom « ESCALOL 507 » par ISP, Glyceryl PABA, PEG-25 PABA vendu sous le nom « UVINUL P25 » par BASF,
- les dérivés salicyliques : Homosalate vendu sous le nom « EUSOLEX HMS » par RONA/EM INDUSTRIES, Ethylhexyl Salicylate vendu sous le nom « NEO HELIOPAN OS » par HAARMANN et REIMER, Dipropyleneglycol Salicylate vendu sous le nom « DIPSAL » par SCHER, TEA Salicylate, vendu sous le nom « NEO HELIOPAN TS » par HAARMANN et REIMER,
- les dérivés du dibenzoylméthane : Butyl Methoxydibenzoylmethane vendu notamment sous le nom commercial « PARSOL 1789 » par HOFFMANN LA ROCHE, Isopropyl Dibenzoylmethane,
- les dérivés cinnamiques : Ethylhexyl Methoxycinnamate vendu notamment sous le nom commercial « PARSOL MCX » par HOFFMANN LA ROCHE, Isopropyl Methoxy cinnamate, Isoamyl Methoxy cinnamate vendu sous le nom commercial « NEO HELIOPAN E 1000 » par HAARMANN et REIMER, Cinoxate, DEA Methoxycinnamate, Diisopropyl Methylcinnamate, Glyceryl Ethylhexanoate Dimethoxycinnamate,
- les dérivés de β,β'-diphénylacrylate: Octocrylene vendu notamment sous le nom commercial « UVINUL N539 » par BASF, Etocrylene, vendu notamment sous le nom commercial « UVINUL N35 » par BASF,
- les dérivés de la benzophénone : Benzophenone-1 vendu sous le nom commercial « UVINUL 400 », par BASF, Benzophenone-2 vendu sous le nom commercial « UVINUL D50 » par BASF, Benzophenone-3 ou Oxybenzone, vendu sous le nom commercial « UVINUL M40 » par BASF, Benzophenone-4 vendu sous le nom commercial « UVINUL MS40 » par BASF, Benzophenone-5, Benzophenone-6 vendu sous le nom commercial « HELISORB 11 » par NORQUAY, Benzophenone-8 vendu sous le nom commercial « SPECTRA-SORB UV-24 » PAR AMERICAN CYANAMID, Benzophenone-9 vendu sous le nom commercial« UVINUL DS-49» par BASF, Benzophenone-12,
- les dérivés du benzylidène camphre: 3-Benzylidene camphor, 4-Methylbenzylidene camphor vendu sous le nom « EUSOLEX 6300 » par MERCK, Benzylidene Camphor Sulfonic Acid, Camphor Benzalkonium Methosulfate, Terephthalylidene Dicamphor Sulfonic Acid, Polyacrylamidomethyl Benzylidene Camphor,
- les dérivés du phényl benzimidazole : Phenylbenzimidazole Sulfonic Acid vendu notamment sous le nom commercial « EUSOLEX 232 » par MERCK, Benzimidazilate vendu sous le nom commercial commercial « NEO HELIOPAN AP » par HAARMANN et REIMER,
- les dérivés de la triazine : Anisotriazine vendu sous le nom commercial «TINOSORB S » par CIBA GEIGY, Ethylhexyl triazone vendu notamment sous le nom commercial «UVINUL T150 » par BASF, Diethylhexyl Butamido Triazone vendu sous le nom commercial « UVASORB HEB » par SIGMA 3V,
- les dérivés du phényl benzotriazole : Drometrizole Trisiloxane vendu sous le nom « SILATRIZOLE » par RHODIA CHIMIE ,
- les dérivés anthraniliques : Menthyl anthranilate vendu sous le nom commercial « NEO HELIOPAN MA » par HAARMANN et REIMER,
- les dérivés d'imidazolines: Ethylhexyl Dimethoxybenzylidene Dioxoimidazoline Propionate,
- les dérivés du benzalmalonate : Polyorganosiloxane à fonctions benzalmalonate vendu sous la dénomination commerciale « PARSOL SLX » par HOFFMANN LA ROCHE, et leurs mélanges.

Les filtres UV organiques plus particulièrement préférés sont choisis parmi les composés suivants :
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 4-Methylbenzylidene camphor,
- Benzimidazilate,
- Anisotriazine,
- Ethylhexyl triazone,
- Diethylhexyl Butamido Triazone,
- Methylene bis-Benzotriazolyl Tetramethylbutylphenol,
- Drometrizole Trisiloxane,
et leurs mélanges.

Les filtres inorganiques utilisables dans la composition selon l'invention sont en particulier les nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium. Des agents d'enrobage sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

De façon connue, la composition de l'invention peut contenir également les adjuvants habituels dans les domaines cosmétique et dermatologique, tels que les gélifiants hydrophiles ou lipophiles, les conservateurs, les antioxydants, les solvants, les parfums, les charges, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse ou dans la phase aqueuse. Ces adjuvants, ainsi que leurs concentrations, doivent être tels qu'ils ne nuisent pas aux propriétés avantageuses de l'extrait de bactérie filamenteuse non photosynthétique non fructifiante.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone et la fraction liquide du beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

Comme émulsionnants et coémulsionnants utilisables dans l'invention, on peut citer par exemple les émulsionnants H/E tels que les esters d'acide gras et de polyéthylène glycol, notamment le stéarate de PEG-100, et les esters d'acide gras et de glycérine tels que le stéarate de glycéryle, ainsi que les émulsionnants E/H tels que le poly(méthylcétyl)(diméthyl)méthylsiloxane oxyéthyléné disponible sous la dénomination commerciale ABIL WE09 auprès de la société Degussa Goldschmidt ou le mélange de stéarate d'éthylène glycol acétyle et de tristéarate de glycéryle commercialisé par la société Guardian sous la dénomination commerciale UNITWIX.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme charges qui peuvent être utilisées dans la composition de l'invention, on peut citer par exemple, outre les pigments, la poudre de silice ; le talc ; l'amidon réticulé par l'anhydride octénylsuccinique commercialisé par la société National Starch sous la dénomination DRY FLO PLUS (28-1160) ; les particules de polyamide et notamment celles vendues sous la dénomination ORGASOL par la société Atochem ; les poudres de polyéthylène ; les micro-sphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition ou de la préparation selon l'invention.

### EXEMPLES

### Exemple 1 - Préparation d'un extrait de Vitreoscilla filiformis contenant des lipopolysaccharides

(M. A Apicella, J. McLeod Gritliss, and H. Schneider, 1994 Methods in enzymology, Vol 235, (242-252))

Différentes méthodes permettent d'isoler la fraction d'intérêt contenant les lipopolysaccharides :
- la méthode modifiée phénol-eau (Johnson et Perry, 1975, Can J. Microbial, 22, p 29) décrite dans le paragraphe 1 ;
- la méthode de Darveau et Hancock (1983, J.Bact. 155, p 831) qui utilise le SDS pour solubiliser les lipopolysaccharides, ce qui permet de les séparer du peptidoglycane insoluble, cette méthode est décrite dans le paragraphe 2. Les protéines sont éliminées de la fraction contenant les lipopolysaccharides par une digestion enzymatique (Pronase), la fraction de lipopolysaccharides est ensuite précipitée à l'éthanol.
- la méthode d'extraction utilisant la protéinase K décrite dans le paragraphe 3.

### 1. Technique au phénol modifiée

### 1.1. Préparation des lipopolysaccharides bruts

A 5 g de bactéries *Vitreoscilla filiformis* (ATCC 15551) congelées ou séchées à l'acétone sous forme de poudre sont ajoutés 25 ml de tampon 50 mM phosphate de Na pH 7 contenant 5 mM EDTA, le mélange est agité.
Sont alors réalisées les étapes suivantes :
- ajout de 100 mg de lysozyme, agitation pendant 1 nuit a 4°C, puis incubation à 37°C pendant 20 min ;
- centrifugation pendant 3 min à basse vitesse,
- ajustement du volume à 100 ml avec de tampon 50 mM phosphate de Na pH 7 contenant 20 mM MgCl₂ ;
- ajout de Rnase, Dnase (à 1 µg/ml), incubation pendant 60 min à 37°C puis pendant 60 min a 60°C ;
- la suspension bactérienne est placée dans un bain à 70°C, on lui ajoute un volume égal de phénol 90% (w/v) préchauffé à 70°C,
- elle est refroidie par agitation pendant 15 min dans'un bain glacé,
- centrifugation à 18.000g pendant 15 min à 4°C.
Une interface marquée se fait entre les phases aqueuse et phénolique. La phase aqueuse contient les lipopolysaccharides, après dialyse contre de l'eau, cette phase est lyophilisée.

### 1.2. Purification des lipopolysaccharides bruts

20 à 35 mg des llpopolysaccharldes/ml d'eau distillée sont centrifugés à basse vitesse (1100 g, 5 min). Le surnageant obtenu est alors centrifugé a haute vitesse (105.000g, 16h, 4°C). Le culot est suspendu dans de l'eau, la centrifugation est répétée jusqu'à obtenir des lipopolysaccharides purifiés. Le culot final est resuspendu dans de l'eau et lyophilisé.

### 2. Méthode au SDS

A 500 mg de cellules bactériennes de *Vitreoscilla filiformis* (ATCC 15551) séchées, sont ajoutés 15 ml de tampon 10 mM Tris-HCI pH 8,2 mM MgCl₂, 100 µg/ml Dnase et 25 µg/ml Rnase. On soumet le mélange sous une presse de French 2 fois, 15.000 psi, puis sonication, 2 fois à 6 W, 30 sec.
On applique ensuite les étapes suivantes :
- ajout de Dnase 200 µg final et Rnase 50 µg final, incubation 37°C 2 h,
- ajout de 5 ml 0,5M EDTA dans 10 mM Tris-HCI pH 8, 2,5 ml 20% SDS dissous dans 10 mM Tris-HCI et 2,5 10 mM Tris-HCI pH 8.
Le volume final obtenu est de 25 ml contenant 0,1 M EDTA; 2% SDS et 10mM Tris-HCl pH 9,5, le mélange est vortexé et centrifugé à 50000g pendant 30 min à 20°C.

Le surnageant est décanté. Le sédiment qui contient le peptidoglycane est écarté. La pronase est ajoutée au surnageant a une concentration finale de 200 µM, suit une incubation à 37°C pendant 1 nuit, sous agitation (si un précipité se forme, l'enlever en centrifugeant 1000g 10 min).
Les lipopolysaccharides sont précipités à l'éthanol 95% (v/v) contenant 0,376M de MgCl₂-70°C, puis centrifuger (12.000g, 15 min, 4°C).
Le culot obtenu est suspendu dans 25 ml 2% SDS, 0,1 M EDTA, 10 mM Tris-HCI pH 8, soniqué puis incubé à 85°C, 10 à 30 min. Après refroidissement, la solution est ajustée à pH 9.5.
La Pronase est ajoutée à 25 µg/ml, incubation à 37°C, 1 nuit, sous agitation.
Les lipopolysaccharides sont à nouveau précipités à l'éthanol 95% (v/v) contenant 0,376M de MgCl₂, puis centrifugés (12.000g 15 min 4°C). Pour enlever les cristaux insolubles Mg ²⁺-EDTA, le culot est resuspendu dans 15 ml de tampon 10 mM Tris-HCI pH 8, soniqué et centrifugé (1.000g ,5 min). Le surnageant est alors centrifugé (200.000g, 2h 15°C) en présence de 25 mM MgCl₂. Le culot qui contient les lipopolysaccharides est suspendu dans de l'eau distillée.

### 3. Extraction des lipopolysaccharides utilisant la protéinase K (Zanen and, 1988, FEMS Microbiology Letters 50, 85-88).

A 1,5 g de cellules de *Vitreoscilla filiformis* (ATCC 15551) lyophilisées, sont ajoutés 30 ml de tampon contenant 2% SDS, 5% 2-mercaptoéthanol, 10% glycérol, et 0,25M Tris-HCI pH 6,8.
Le mélange est incubé 15 à 30 min, 100°C, centrifugé (10,000 g, 30 min, 4°C). Le surnageant (20 ml) est récupéré, on ajoute 12 mg de protéinase K, on incube a 60°C pendant 1 h et on précipite les lipopolysaccharides à l'éthanol 95% (v/v) contenant 0,376M de MgCl₂, -20°C pendant une nuit, reprécipiter les lipopolysaccharides à l'éthanol 95% (v/v) dans les mêmes conditions que précédemment, le culot obtenu est suspendu dans 10 ml d'eau, dialysé puis lyophilisé.

### Exemple 2 - Formulations

| Composition 1 - Crème régénératrice | |
|---|---|
| - Extrait de l'exemple 1 (extrait lyophilisé obtenu selon la méthode 3) | 0,1 % |
| - Carbomer 940® (acide polyacrylique réticulé) | 0,3 % |
| - Triéthanolamine | 0,3 % |
| - Acide stéarique | 3,0 % |
| - Alcool cétylique | 2,0 % |
| - Monostéarate de glycérol autoémulsionnable | 3,0 % |
| - Huile de soja | 10,0% |
| - Alcool de lanoline | 2,0% |
| - Myristate d'isopropyle | 4,0 % |
| - 2-éthylhexanoate de cétyle et de stéaryle | 4,0 % |
| - Perhydrosqualène | 3,0 % |
| - Paraffine | 2,0 % |
| - Glycérine | 3,0 % |
| - Conservateurs | 0,3 % |
| - Eau | qsp 100 |

Pour préparer cette crème, on chauffe la phase aqueuse contenant la glycérine, les conservateurs et l'eau à 80°C ; on y disperse le Carbomer 940 qui est ensuite neutralisé par la triéthanolamine. La phase grasse, chauffée et homogénéisée à 80°C, est introduite sous vive agitation dans la phase aqueuse. L'extrait de l'exemple est dispersé dans 10 g d'eau et introduit à 40°C dans la crème, sous agitation. L'ensemble est refroidi jusqu'à température ambiante.
Cette crème est appliquée sur la peau du visage et du cou une à deux fois par jour. Elle permet notamment, après utilisation pendant quelques jours, d'augmenter la régénération de l'épiderme et de donner un aspect de peau plus jeune.

| Composition 2 - Gel pour le soin du visage | |
|---|---|
| Extrait de l'exemple 1 (extrait lyophilisé obtenu selon la méthode 3) | 0,05 % |
| Hydroxypropylcellulose (Klucel H vendu par la société Hercules) | 1,00 % |
| Antioxydant | 0,05 % |
| Isopropanol | 40,00 % |
| Conservateur | 0,30 % |
| Eau | qsp 100 % |

Ce gel est obtenu par mélange des constituants dans l'eau avec l'ajout, en dernier lieu du gélifiant.
Comme pour la composition 1, il peut être appliqué deux fois par jour, il est particulièrement adapté à une application le matin car ne laisse pas la peau grasse.

| Composition 3 - Crème régénératrice | |
|---|---|
| - Extrait de l'exemple 1 (extrait lyophilisé obtenu selon la méthode 3) | 1,0 % |
| - Conservateurs | 0,85 % |
| - Alcool | 5,0 % |
| - Tocopheryl acetate | 1,0 % |
| - Disodium EDTA | 0,05 % |
| - PEG-20 methyl glucose sesquistearate | 2,0 % |
| - Glycérine | 7,0 % |
| - Acrylate polymer | 0,25 % |
| - Cholesterol | 0,1 % |
| - Cyclohexasiloxane | 3,5 % |
| - Squalane | 9,5 % |
| - eau et extrait de Fagus Sylvatica | 2,0 % |
| - Céramide | 0,05 % |
| - Ammonium polyacryloyldimethyl taurate | 2,2 % |
| - Hydrolysed lupine protein | 1,0 % |
| - Huiles végétales | 6,0 % |
| - Polycaprolactone and solanum lycopersicum (Tomato) extract (lycopène) | 1,0% |
| - Divinyldimethicone/dimethicone copolymer (and) C12-13 pareth-3 (and) C12-13 pareth-23 | 2,0 % |
| - Eau | qsp 100 |

Cette crème est préférentiellement destinée à être appliquée quotidiennement le soir après nettoyage de la peau. Cette crème permet rapidement à la peau d'être mieux hydratée et plus souple, elle rend le teint lumineux et uniforme et a un effet tenseur qui estompe les rides et ridules et lisse la peau.

| Composition 4 - Fluide régénérant pour le jour | |
|---|---|
| - Extrait de l'exemple 1 (extrait lyophilisé obtenu selon la méthode 3) | 1,0 % |
| - Octyldodecanol | 0,1 % |
| - Conservateurs | 0,75 % |
| - Tocopheryl acetate | 1,0 % |
| - PEG-20 methyl glucose sesquistearate | 3,0 % |
| - Sodium hyaluronate | 0,1 % |
| - Glycerin | 7,0 % |
| - Cyclohexasiloxane | 9,5 % |
| - eau et extrait de Fagus Sylvatica | 1,0 % |
| - Ammonium polyacryloyldimethyl taurate | 1,0 % |
| - Cyclopentasiloxane (and) dimethiconol | 7,5 % |
| - Hydrolysed lupine protein | 1,0 % |
| - Polycaprolactone and solanum lycopersicum (Tomato) extract (lycopène) | 1,0% |
| - Huiles végétales | 3,0 |
| - Divinyldimethicone/dimethicone copolymer (and) C12-13 pareth-3 (and) | |
| C12-13 pareth-23 | 2,0 % |
| - water | qsp 100 |

Ce fluide peut être appliqué quotidiennement matin et soir. Comme pour la crème précédente, ce fluide améliore l'aspect visuel de la peau et du teint grâce à une meilleure hydratation et un effet tenseur.

| Composition 5 - Crème de soin de l'érythème solaire (émulsion huile-dans-eau) | |
|---|---|
| Extrait de l'exemple 1 (extrait lyophilisé obtenu selon la méthode 3) | 0,75 % |
| Stéarate de glycérol | 2,00 % |
| Polysorbate 60 (Tween 60 vendu par la société ICI) | 1,00 % |
| Acide stéarique | 1,40 % |
| Acide glycyrrhétinique | 2,00 % |
| Triéthanolamine | 0,70 % |
| Carbomer | 0,40 % |
| Fraction liquide du beurre de karité | 12,00 % |
| Huile de tournesol | 10,00 % |
| Lycopène | 0,05 % |
| Parfum | 0,50 % |
| Conservateur | 0,30 % |
| Eau | qsp 100% |

Cette crème devra être appliquée deux fois par jour pendant au moins deux jours, puis une fois par jour jusqu'à complète disparition de l'érythème. Grâce à une telle application, la peau retrouve plus rapidement son aspect normal.

| Composition 6 - Filtre solaire | |
|---|---|
| Extrait de l'exemple 1 (extrait lyophilisé obtenu selon la méthode 3) | 3,5 % |
| Mélange d'alcool cetylstearylique | |
| et d'alcool cetylstearylique oxyethylene (33 OE) 80/20 | 7,0 % |
| Mélange de mono et de distearate de glycerol | 2,0 % |
| alcool cetylique | 1,5 % |
| poly dimethylsiloxane | 1,5% |
| huile de vaseline | 15,0% |
| butyl methoxydibenzoylmethane | 3,0 % |
| octocrylene | 7,0 % |
| glycerine | 20,0 % |
| eau demineralisee | qsp 100% |

L'application de cette crème devra précéder l'exposition au soleil et être renouvelée toute les deux heures. Une telle application prévient les dommages pouvant être provoqués par le soleil et accélère la réparation de ces éventuels dommages.

| Composition 7 - Composition pour préparer la peau au soleil | |
|---|---|
| (les ingrédients sont indiqués sous leurs dénominations CTFA) | |
| Extrait de l'exemple 1 (extrait lyophilisé obtenu selon la méthode 3) | 0,5 % |
| Conservateurs | 1,35 % |
| Sodium citrate | 0,035 % |
| PEG-40 | 1,25 % |
| Pentaerythrityl tetraethylhexanoate | 4 % |
| Glycerin | 7 % |
| Sorbitan tristearate | 0,3 % |
| Prunus Armeniaca (abricot) Kernel oil | 2 % |
| Cetyl alcohol | 0,7 |
| Propylene glycol | 2 % |
| Triethanolamine | 0,4 % |
| Cyclohexasiloxane | 2 % |
| Carbomer | 0,75 % |
| Tocopherol | 1 % |
| Silica | 2 % |
| Ascorbyl glucoside | 0,1 % |
| Eau - Titanium dioxide - silica - alumina | 3 % |
| Polycaprolactone - bêta carotène | 5 % |
| Eau qsp | 100 % |

L'application quotidienne de cette composition au moins une semaine avant l'exposition solaire diminue les risques d'érythèmes, dé brûlure et d'oedème (en particulier, si la peau est protégée avec la composition 4 pendant l'exposition solaire) et permet l'obtention d'un bronzage de meilleure qualité et plus durable.

### Exemple 3 - Mesure de l'augmentation de la synthèse de MnSOD

### I. Matériel et méthodes.

### - Cultures cellulaires et traitement :

Les études sont réalisées sur fibroblastes normaux humains et sur kératinocytes épidermiques humains normaux en culture.
Les fibroblastes dermiques sont cultivés en milieu DMEM (Life Technology, ref. 21969035) contenant de la L-glutamine (2 mM, Life Technology, ref. 25030024), de la pénicilline à 50 UI/ml et streptomycine à 50 µg/ml (Life Technology, ref. 15070063) et du sérum de veau foetal à 1% (v/v, Life Technology, ref. 10106151).
Les kératinocytes sont cultivés en milieu SFM sans extrait pituitaire et EGF.
L'extrait de *Vitreoscilla filiformis* obtenu en appliquant la méthode 3 de l'exemple 1 a été appliqué sur les cellules à la concentration de 0.1 % (P/V). Le temps de contact a été de 24 ou 48 heures.

### - CDNA array :

La méthodologie utilisée est celle préconisée par Clontech (Palo Alto, USA). L'extraction/purification de l'ARN total de chaque culture a conduit à l'isolement de quantités d'ARN total de l'ordre de 100 à 150 µg. Les solutions d'ARN total sont traitées par la DNAse I pour éliminer toute trace d'ADN contaminant, selon les recommandations du fournisseur. La qualité de l'ARN a ensuite été vérifiée sur gel d'agarose et les solutions d'ARN ont été ajustées à 1 µg/ml.
L'étape suivante a été la purification des ARN messagers (ARNm) par hybridation des extrémités poly(A) des ARNm à des amorces oligo(dT) biotinylées et capture sélective sur billes de streptavidine, selon le protocole Atlaspure (Clontech). Les sondes ADN marquées au ³³P ont été réalisées par reverse-transcription des ARNm liés sur billes de poly(dT), à l'aide d'un pool de primers spécifiques des séquences immobilisées sur les « arrays », en présence de (α³³P)-dATP. Cette étape a utilisé les réactifs et le protocole préconisé par Clontech. Les sondes marquées ont été purifiées par chromatographie sur colonne d'exclusion et la qualité et l'équivalence des sondes marquées a été évaluée par comptage en scintillation liquide.
Des membranes de cDNA array comportant 1176 gènes ont été pré-traitées puis les cDNAs immobilisés sur chaque membrane ont été hybridés (68°C, une nuit) aux sondes marquées correspondantes. Les filtres ont ensuite été lavés extensivement et placés dans des sacs plastiques individuels pour analyse. L'analyse a eu lieu par quantification directe de la radioactivité des spots à l'aide d'un phospholmager Cyclone (Packard). Les résultats sont exprimés en unités relatives d'expression (RE, radioactivité du spot correspondant à chaque gène, corrigé du bruit de fond et des différences d'intensité de marquage des sondes).

### - RT-Q-PCR :

Les couples de primer utilisés dans cette étude sont la Mn+ superoxide dismutase 2 precursor (taille du fragment amplifié : 259 pb) et la cytosolic superoxide dismutase 1 (taille du fragment amplifié : 298 bp).
Les ARN totaux sont extraits à l'aide de Tri-Reagent selon le protocole préconisé par le fournisseur. Elle est suivie d'une nouvelle extraction par du chloroforme et d'une précipitation à l'isopropanol. Les traces d'ADN potentiellement contaminantes sont éliminées par traitement avec le système DNA-free (Ambion). La réaction de réverse-transcriptase est ensuite réalisée en présence de l'amorce oligo(dT) et de l'enzyme Superscript II (Gibco). Cette étape est suivie d'une quantification, par fluorescence, de l'ADNc synthétisé et ajustement des concentrations à 150 ng/ml. Une nouvelle quantification de chaque ADNc, après dilution finale, est réalisée avant la réaction de PCR.

Les réactions de PCR (polymérase chain réactions) ont été réalisées par PCR quantitative avec le système « Light Cycler » (Roche Molecular Systems Inc.) et selon les procédures recommandées par le fournisseur. Le mélange réactionnel (10 µl final) introduit dans les capillaires d'un thermocycleur est composé de 2.5 µl d'ADNc, des amorces des deux marqueurs, du mélange réactionnel (Roche) contenant l'enzyme taq DNA polymérase, le marqueur SYBR Grenn I. Les conditions de la PCR sont les suivante : activation de 10 min. à 95°C, réactions de PCR en 40 cycles, fusion 5 sec à 95 °C puis 5 sec. à 60 °C.
L'analyse de fluorescence dans l'ADN amplifié est mesurée en continu au cours des cycles de PCR. La valeur moyenne de l'expression relative (RE) est exprimée en Unités Arbitraires (UA) calculées à partir des valeurs de cycles de deux PCRs indépendantes selon la formule suivante : (1/2 ^{nombre de cycles}) x 10⁶.
Les résultats d'expression de SOD sont comparées à celle de l'actine afin de prendre en compte les éventuels écarts de concentration cellulaire dans les deux populations cellulaires.

### - Etude en cytométrie de flux :

Les cellules sont pré-cultivées à forte densité pendant 48 heures puis traitées ou non par le produit à l'essai pendant 24 ou 48 heures. Les cellules sont trypsinisées puis rincées par une solution PBS/2 % SVF. Les cellules sont transférées en tubes Eppendorf et centrifugées 5' à 1500 rpm. Les cellules sont fixées par une solution PBS-formol à 4 % final, 30' à température ambiante et à l'obscurité. elles sont ensuite perméabilisées à l'aide d'une solution 0.1 % Triton-X100 / 0.1 % citrate. Le marquage des cellules est réalisé en présence d'anticorps anti-MnSOD (TEBU SOD-110) et anti-Cu/Zn SOD (TEBU SOD-100) selon les indications du fournisseur. Le marquage secondaire est réalisé à l'aide de l'anticorps secondaire-FITC (TEBU L 42001) selon les indications du fournisseur. L'analyse des échantillons est réalisée par cytométrie (cytomètre FACSCAN, logiciel Cell Quest; Becton-Dickinson) sur la population totale. Les statistiques sont réalisées sur 10 000 cellules de chaque échantillon. Les résultats sont exprimés en intensité de fluorescence (IF) correspondant à la quantité relative de chaque marqueur par cellule dans une population totale de 10 000 cellules analysées.

### II. Résultats

### 2.1) Etude de cDNA macroarray

### 2.1.1) sur culture de kératinocytes humains

| Gènes | Population Témoin | Population exposée à l'extrait | Comparaison de la population témoin et de la population exposée à l'extrait (en %) |
|---|---|---|---|
| Mn+ SOD2 precursor (SOD2) | 1.3 | 3.3 | 244 |
| Cytosolic SOD1 (SOD1) | 4.9 | 6.8 | 138 |

On constate que la population cellulaire constituée de kératinocytes exposée à l'extrait de bactérie filamenteuse non photosynthétique non fructifiante contient significativement plus d'ARNm de SOD2 que la population témoin alors que la quantité d'ARNm de SOD1 n'est que modérément augmentée.

### 2.1.2) sur culture de fibroblastes humains

| Gène | Population Témoin | Population exposée à l'extrait | Comparaison de la population témoin et de la population exposée à l'extrait (en %) |
|---|---|---|---|
| Mn+ SOD 2 précursor (SOD2) | 1.9 | 9.3 | 481 |

De la même façon que pour les kératinocytes, une population cellulaire constituée de fibroblaste exposée à un extrait de bactérie filamenteuse non photosynthétique non fructifiante contient près de 5 fois plus d'ARNm de SOD2 que la population témoin.

### 2.2) Etude de PCR

Les résultats ci-dessous sont ceux obtenus sur culture de kératinocytes humains.

### 2.2.1) résultats sur la superoxyde dismutase cytosolique 1 (SOD1)

| Traitement | Cycles Actine | Cycles SOD1 | RE*Actine (UA) | RE*SOD1 (UA) | SOD1/ Actine | % Témoin |
|---|---|---|---|---|---|---|
| Témoin | 16.12 | 20.12 | 13.99 | 0.93 | 6.64 10-2 | 100 |
| | 16.13 | 19.96 | | | | |
| Extrait VF | 16.23 | 20.02 | 12.88 | 0.94 | 7.28 10-2 | 110 |
| | 16.26 | 20.03 | | | | |

| | | | | | | |
|---|---|---|---|---|---|---|
| (VF : *Vitreoscilla filiformis)* | | | | | | |

Ces résultats confirment ceux observés au point 2.1.1., c'est à dire que l'exposition d'une population de kératinocytes à l'extrait de bactérie préparé selon la méthode 3 ne provoque pas d'induction transcriptionnelle de SOD1.

### 2.2.2) résultats sur la Mn+ superoxide dismutase (SOD2)

| Traitement | Cycles Actine | Cycles SOD2 | RE*Actine (UA) | RE*SOD2 (UA) | SOD2/ Actine | % Témoin |
|---|---|---|---|---|---|---|
| Témoin | 20.78 | 26.45 | 0.55 | 0.01 | 2.37 10-2 | 100 |
| | 20.80 | 25.97 | | | | |
| Extrait VF | 20.79 | 23.65 | 0.51 | 0.09 | 1.79 10-2 | 755 |
| | 21.02 | 23.16 | | | | |

| | | | | | | |
|---|---|---|---|---|---|---|
| (VF : *Vitreoscilla filiformis*) | | | | | | |

Ces résultats confirment également ceux du point 2.1.1. : la quantité d'ARNm codant pour la SOD2 est très supérieure dans la population cellulaire qui a été exposée à l'extrait bactérien.

### 2.3) Etude de cvtométrie de flux

Les résultats ci-dessous sont ceux obtenus sur culture de kératinocytes humains.

### 2.3.1) résultats sur la superoxide dismutase cytosolique 1 (SOD1)

Quantité relative de Cu/Zn SOD dans des kératinocytes témoins et dans des kératinocytes traités par l'extrait (0.1 %, P/V) pendant 24 heures.

| Traitement | IF | Ecart type | Nombre d'échantillon | % | p |
|---|---|---|---|---|---|
| Témoin | 180.9 | 15.3 | 3 | 100 | - |
| Extrait VF | 217.8 | 7.3 | 3 | 120 | P<0.01 |

| | | | | | |
|---|---|---|---|---|---|
| (VF : *Vitreoscilla filiformis)* | | | | | |

On observe que bien que l'exposition à l'extrait VF n'induit pas une transcription d'ARNm de SOD1, son expression est statistiquement augmentée (+20%).

### 2.3.2) résultats sur la Mn superoxide dismutase (SOD2)

Quantité relative de MnSOD dans des kératinocytes témoins et traités par l'extrait (0.1 %) pendant 48 heures.

| Traitement | IF | Ecart type | Nombre d'échantillon | % | p |
|---|---|---|---|---|---|
| Témoin | 20.2 | 2.6 | 3 | 100 | - |
| Extrait VF | 28.1 | 1.8 | 3 | 139 | P<0.01 |

| | | | | | |
|---|---|---|---|---|---|
| (VF : *Vitreoscilla filiformis*) | | | | | |

Ces essais aussi montre une augmentation de la concentration cellulaire en SOD2 (+39%) après exposition à l'extrait VF.

L'observation de ces essais conduit à la conclusion que les fibroblastes exposés en présence de l'extrait obtenu dans l'exemple 1 expriment fortement les ARNs messagers codant pour la MnSOD (2.1. et 2.2.) et cette induction a pu être confirmée par l'observation d'une augmentation de la présence protéique de MnSOD (2.3.), en particulier au niveau des mitochondries (visualisé par observations microscopiques).

## Revendications

1. Utilisation cosmétique d'un isolat de lipopolysaccharides de bactérie filamenteuse non photosynthétique non fructifiante dans une composition renfermant un milieu cosmétiquement acceptable, en tant qu'agent augmentant la synthèse endogène de superoxyde dismutase,:
- pour prévenir et/ou lutter contre les effets néfastes de la pollution sur la peau,
- pour prévenir et/ou traiter le teint terne,
- pour prévenir et/ou traiter la déshydratation cutanée,
- pour prévenir et/ou limiter et/ou éliminer la péroxydation des lipides cutanés,
- pour préparer la peau à l'exposition solaire,
- pour atténuer et/ou réparer les rougeurs et/ou les irritations cutanées et/ou les sensations d'échauffement de la peau provoquées par une exposition solaire ou
- lorsque ladite composition est appliquée sur les ongles, pour revitaliser les ongles, via la prévention et/ou la limitation de la formation de radicaux libres et/ou l'élimination des radicaux libres présents dans les cellules.

2. Utilisation selon la revendication 1, **caractérisée en ce que** la bactérie filamenteuse non photosynthétique non fructifiante est *Vitreoscilla filiformis.*

3. Utilisation selon l'une quelconque des revendications précédentes, **caractérisée en ce que** l'extrait de bactérie filamenteuse non photosynthétique non fructifiante est associé à au moins un agent antioxydant.

4. Utilisation selon la revendication 3, **caractérisée en ce que** l'agent antioxydant est le lycopène.

5. Utilisation selon la revendication 3 ou 4, **caractérisée en ce que** l'agent antioxydant est une superoxyde dismutase.

6. Utilisation selon la revendication 1, pour prévenir et/ou limiter et/ou éliminer les mauvaises odeurs corporelles, sous réserve que ledit isolat soit utilisé pour prévenir et/ou limiter et/ou éliminer la péroxydation des lipides cutanés.

7. Utilisation selon l'une quelconque des revendications précédentes, **caractérisée en ce que** la composition est adaptée à une application topique.

8. Utilisation selon l'une quelconque des revendications 1 à 5 et 7, **caractérisée en ce que** la composition est un tonique capillaire pour revitaliser le cuir chevelu et améliorer l'aspect de la chevelure.

9. Utilisation selon l'une quelconque des revendications précédentes, **caractérisée en ce que** la composition contient de 0,001 à 10 % en poids d'extrait sec de bactérie filamenteuse non photosynthétique non fructifiante par rapport au poids total de la composition.

10. Composition renfermant, dans un milieu physiologiquement acceptable, au moins un isolat de lipopolysaccharides de bactérie filamenteuse non photosynthétique non fructifiante et de 10⁻⁷ % à 10⁻³ % en poids de lycopène pur par rapport au poids total de la composition.

## Claims

1. Cosmetic use of a lipopolysaccharide isolate of a nonfruiting nonphotosynthetic filamentous bacterium in a composition containing a cosmetically acceptable medium, as an agent increasing the endogenous synthesis of superoxide dismutase:
- for preventing and/or combating the harmful effects of pollution on the skin,
- for preventing and/or treating dull complexion,
- for preventing and/or treating skin dehydration,
- for preventing and/or limiting and/or eliminating the peroxidation of skin lipids,
- for preparing the skin for solar exposure,
- for attenuating and/or repairing the redness and/or skin irritations and/or sensations of overheating of the skin caused by solar exposure or
- when the said composition is applied to the nails, for revitalizing the nails,
through preventing and/or limiting the formation of free radicals and/or eliminating the free radicals present in cells.

2. Use according to Claim 1, **characterized in that** the nonfruiting nonphotosynthetic filamentous bacterium is *Vitreoscilla filiformis.*

3. Use according to either of the preceding claims, **characterized in that** the extract of the nonfruiting nonphotosynthetic filamentous bacterium is combined with at least one antioxidant.

4. Use according to Claim 3, **characterized in that** the antioxidant is lycopene.

5. Use according to Claim 3 or 4, **characterized in that** the antioxidant is superoxide dismutase.

6. Use according to Claim 1, for preventing and/or limiting and/or eliminating bad body odours, with the proviso that the said isolate is used for preventing and/or limiting and/or eliminating the peroxidation of skin lipids.

7. Use according to any one of the receding claims, **characterized in that** the composition is suited to topical application.

8. Use according to any one of Claims 1 to 5 and 7, **characterized in that** the composition is a hair tonic for revitalizing the scalp and improving the appearance of the hair.

9. Use according to any one of the preceding claims, **characterized in that** the composition contains from 0.001 to 10% by weight of dry extract of a nonfruiting nonphotosynthetic filamentous bacterium relative to the total weight of the composition.

10. Composition containing, in a physiologically acceptable medium, at least one lipopolysaccharide isolat of a nonfruiting nonphotosynthetic filamentous bacterium and from 10⁻⁷% to 10⁻³% by weight of pure lycopene relative to the total weight of the composition.

## Patentansprüche

1. Kosmetische Verwendung eines Isolats von Lipopolysacchariden eines filamentösen, nicht zur Photosynthese befähigten, nicht fruktifizierenden Bakteriums in einer Zusammensetzung, die ein kosmetisch akzeptables Medium enthält, als Stoff, der die endogene Synthese der Superoxid-Dismutase steigert:
- um den schädlichen Wirkungen der Umweltbelastung auf die Haut vorzubeugen und/ oder sie zu bekämpfen;
- um glanzlosem Teint vorzubeugen und/oder ihn zu behandeln;
- um der Dehydratisierung der Haut vorzubeugen und/oder sie zu behandeln;
- um der Peroxidation von Hautlipiden vorzubeugen und/ oder sie zu begrenzen und/oder sie zu beseitigen;
- um die Haut auf eine Sonnenexposition vorzubereiten;
- um Rötungen und/oder Hautirritationen und/oder das Gefühl der Aufheizung der Haut, die durch eine Sonnenexposition hervorgerufen werden, zu lindern und/oder zu beheben; oder
- die Nägel zu revitalisieren, wenn die Zusammensetzung auf die Nägel aufgebracht wird;
via Verhütung und/oder Begrenzung der Bildung freier Radikale und/ oder Beseitigung der in den Zellen vorhandenen freien Radikale.

2. Verwendung nach Anspruch 1, **dadurch gekennzeichnet, dass** es sich bei dem filamentösen, nicht zur Photosynthese befähigten, nicht fruktifizierenden Bakterium um *\*/*itreoscilla filiformis* handelt.

3. Verwendung nach einem der vorhergehenden Ansprüche, **dadurch gekennzeichnet, dass** der Extrakt eines filamentösen, nicht zur Photosynthese befähigten, nicht fruktifizierenden Bakteriums mit mindestens einem Antioxidationsmittel kombiniert wird.

4. Verwendung nach Anspruch 3, **dadurch gekennzeichnet, dass** es sich bei dem Antioxidationsmittel um Lycopin handelt.

5. Verwendung nach Anspruch 3 oder 4, **dadurch gekennzeichnet, dass** das Antioxidationsmittel eine Superoxid-Dismutase ist.

6. Verwendung nach Anspruch 1, um unangenehmen Körpergerüchen vorzubeugen und/ oder sie zu begrenzen und/oder sie zu beseitigen, mit der Maßgabe, dass das Isolat verwendet wird, um der Peroxidation von Hautlipiden vorzubeugen und/oder sie zu begrenzen und/oder sie zu beseitigen.

7. Verwendung nach einem der vorhergehenden Ansprüche, **dadurch gekennzeichnet, dass** die Zusammensetzung für eine topische Anwendung geeignet ist.

8. Verwendung nach einem der Ansprüche 1 bis 5 und 7, **dadurch gekennzeichnet, dass** es sich bei der Zusammensetzung um ein Haartonikum zur Revitalisierung der Kopfhaut und Verbesserung des Aussehens des Haares handelt.

9. Verwendung nach einem der vorhergehenden Ansprüche, **dadurch gekennzeichnet, dass** die Zusammensetzung 0,001 bis 10 Gew.-% Trockenextrakt des filamentösen, nicht zur Photosynthese befähigten, nicht fruktifizierenden Bakteriums, bezogen auf das Gesamtgewicht der Zusammensetzung, enthält.

10. Zusammensetzung, die in einem physiologisch akzeptablen Medium ein Isolat von Lipopolysacchariden eines filamentösen, nicht zur Photosynthese befähigten, nicht fruktifizierenden Bakteriums und 10⁻⁷ bis 10⁻³ Gew.-% Lycopin, bezogen auf das Gesamtgewicht der Zusammensetzung, enthält.
